(19) **Europäisches Patentamt**

**European Patent Office**

**Office européen des brevets**

(11) Veröffentlichungsnummer: **0 325 944**
**A1**

(12) **EUROPÄISCHE PATENTANMELDUNG**

(21) Anmeldenummer: **89100396.4**

(22) Anmeldetag: **11.01.89**

(30) Priorität: **27.01.88 DE 8800910 U**
**12.10.88 DE 3834698**

(43) Veröffentlichungstag der Anmeldung:
**02.08.89 Patentblatt 89/31**

(84) Benannte Vertragsstaaten:
**ES GR**

(51) Int. Cl.⁴: **D06F 39/02 , D06M 21/02**

(71) Anmelder: **Henkel Kommanditgesellschaft auf Aktien**
**Henkelstrasse 67**
**D-4000 Düsseldorf 13(DE)**

(72) Erfinder: **Weber, Rudolf**
**Am Nettchesfeld 51**
**D-4000 Düsseldorf 13(DE)**

(74) Vertreter: **Rieder, Hans-Joachim, Dr. et al**
**Corneliusstrasse 45 Postfach 11 06 42**
**D-5600 Wuppertal 11(DE)**

(54) **Vorrichtung zur Aufnahme und Abgabe eines Behandlungsmittels.**

㊸ Die Erfindung betrifft eine Vorrichtung zur Aufnahme und Abgabe eines Behandlungsmittels, insbesondere Flüssigwaschmittels, bestehend aus einem das Behandlungsmittel aufnehmenden Träger. Sie schlägt für einen hohen Wirkungsgrad und eine Wäscheschonung sowie gute Handhabbarkeit vor, daß der als Speicher ausgebildete Träger mindestens eine Lage einer einseitig mit einer flüssigkeitsundurchlässigen Folie (3) versehenen offenporigen, flexiblen Zellstruktur (1) aufweist.

Fig. 1

EP 0 325 944 A1

## Vorrichtung zur Aufnahme und Abgabe eines Behandlungsmittels

Die Erfindung betrifft eine Vorrichtung zur Aufnahme und Abgabe eines Behandlungsmittels, bestehend aus einem das Behandlungsmittel aufnehmenden Träger.

Als Wäschebehandlungsmittel ausgebildetes Behandlungsmittel wird üblicherweise über die in den Waschmaschinen vorhandenen Einspülvorrichtungen dosiert. Dabei kann es bei flüssigem Behandlungsmittel vorkommen, daß ein Teil des Behandlungsmittels zu Beginn des Waschvorganges in den Laugenstutzen der Waschmaschine läuft und somit für den Waschprozeß nicht mehr vollständig aktiviert werden kann. Ähnliches gilt für die Zugabe von Weichmachungsmitteln zur zu behandelnden Wäsche.

Bezüglich Flüssig-Waschmittel ist man dazu übergegangen, dieses in einen als Behälter ausgebildeten Speicher zu füllen, der zu Beginn des Waschvorganges zusammen mit der Wäsche in die Trommel der Waschmaschine gestellt wird. Bei nicht einwandfreier Handhabung kann es jedoch vorkommen, daß durch die Trommelbewegung der Behälter seinen Inhalt über eine Auslaßöffnung so ungünstig abgibt, daß dieser - wie zuvor beschrieben - direkt in den Laugenstutzen fließt und somit ebenfalls für den Waschprozeß nicht mehr vollständig zur Verfügung steht. Da der Behälter aus relativ festem Kunststoffmaterial gefertigt ist und während des gesamten Waschvorganges zusammen mit der Wäsche umgewälzt wird, stuft die Hausfrau wegen der auftretenden Reibung des Behälters mit den Wäschestücken diesen als nicht besonders textilfreundlich ein. Überdies kann durch das Umwälzen in der Waschmaschinentrommel eine störende Geräuschentwicklung auftreten.

Aus der DE-PS 21 50 586 ist eine Vorrichtung zum Konditionieren von Textilien mit einem Wäschebehandlungsmittel der eingangs genannten Art bekannt, bei der das Konditionierungsmittel auf einen formstabilen Träger aus synthetischem Kunststoffmaterial als Oberflächenbeschichtung aufgebracht ist. Die Konditionierung der Textilien erfolgt dadurch, daß die Vorrichtung zusammen mit diesen umgewälzt wird oder die Textilien beim Umwälzen an der fest angeordneten Vorrichtung entlangstreifen. Mit dem Abtrag des Konditionierungsmittels ist die Vorrichtung verbraucht und nicht mehr mit Wirkung einsetzbar. Durch die Oberflächenbeschichtung ist das Speichervolumen für das Konditionierungsmittel gering, und überdies führt die Unelastizität zu einer starken Textilbeanspruchung.

Des weiteren ist es zur Vorbehandlung von stark verschmutzten Stellen bzw. Flecken an Textilien bekannt, breiiges Behandlungsmittel mit Schwämmen ect. an den entsprechenden Stellen unter Reiben oder anderen mechanischen Manipulationen aufzutragen.

Der Erfindung liegt die Aufgabe zugrunde, eine Vorrichtung der eingangs genannten Art zu schaffen, die ein großes Speichervermögen für das Behandlungsmittel besitzt, textilfreundlich sowie handhabungstechnisch günstig ist und einen häufig wiederkehrenden Einsatz gestattet.

Diese Aufgabe wird erfindungsgemäß dadurch gelöst, daß der als Speicher ausgebildete Träger mindestens eine Lage einer einseitig mit einer flüssigkeitsundurchlässigen Folie versehenen offenporigen, flexiblen Zellstruktur aufweist.

Zufolge derartiger Ausgestaltung ist eine gattungsgemäße Vorrichtung von breitem Einsatzspektrum geschaffen. Sie bietet sich z.B. für die Dosierung eines flüssigen Wäschebehandlungsmittels, insbesondere Flüssig-Waschmittels an. Dieses wird vor Beginn des Waschvorganges aus einer Vorratsflasche oder einem Dosierbehälter heraus auf die Zellstruktur gegossen, wozu diese mit ihrer Folie nach unten vorzugsweise auf eine ebene Unterlage gelegt wird. Durch die Saugwirkung der Zellstruktur wird das flüssige Wäschebehandlungsmittel aufgenommen und "gebunden". Die Folie verhindert nun, daß das flüssige Wäschebehandlungsmittel durch die Zellstruktur hindurchtritt und somit teilweise verlorengeht bzw. die Unterlage benetzt. Die Bindungswirkung einer entsprechenden Zellstruktur ist so groß, daß nach der Verteilung des flüssigen Wäschebehandlungsmittels auch dann kein Herauslaufen des Mittels erfolgt, wenn die Zellstrukturlage zum Einbringen in die Trommel der Waschmaschine senkrecht gehalten wird. Danach wird die in dieser Weise getränkte Zellstruktur für den Waschvorgang zwischen die zu behandelnden Textilien gelegt. Die Saugwirkung der Zellstruktur verhindert, daß Wäschebehandlungsmittelanteile durch Einlaufen in den Laugenstutzen der Waschmaschine verlorengehen. Da es sich bei der erfindungsgemäßen Vorrichtung um eine saugfähige, weiche Zellstruktur handelt, die zu den zu behandelnden Textilien hinzugegeben wird, ist zum einen eine Geräuschentwicklung beim Waschen vermieden und zum anderen ist ein textilfreundliches Verhalten gewährleistet, da keine Reibung an einer harten Struktur wie beim Stand der Technik vorliegt. Von besonderer Bedeutung ist, daß die Übertragung des Wäschebehandlungsmittels an die zu behandelnden Textilien beim Waschvorgang im wesentlichen erst dann erfolgt, wenn Wasser hinzutritt, d.h. wenn die zunächst trockenen Textilien durch Wasserzulauf beim Beginn des Waschvorganges benetzt werden. Wird die erfindungsgemäße Vorrichtung erst nach der zu behandelnden Wäsche in die Waschmaschinentrommel mit der Folie nach unten eingebracht, so ist auch der direkte Kontakt zwischen Wäsche und Wäschebehandlungsflüssigkeit vermieden, der zu Flecken führen kann. Die offenporige Zellstruktur kann die Wäschebehandlungsflüssigkeit auf ihrem gesamten Querschnitt speichern, so daß eine große Menge aufgenommen werden kann. Da vor jedem Waschvorgang oder dergleichen die Wäschebehandlungsflüssigkeit auf die erfindungsgemäße Vorrichtung aufgebracht wird, ist eine laufende Wiederverwertbarkeit des Erfindungsgegenstandes gegeben.

Die Vorrichtung ist nicht auf die Verwendung von Flüssig-Waschmittel beschränkt. Vielmehr ist der Einsatz beliebiger, flüssiger Wäschebehandlungsmittel denkbar. So liegt es auch im Bereich der Erfindung, als Wäschebehandlungsflüssigkeit einen Weichmacher (Weichspüler) einzusetzen, der, auf die Zellstruktur aufgebracht, zu den Textilien, z.B. im Trockner, gegeben wird. Da die Vorrichtung Flüssig-Waschmittel gut bindet, kann man damit auch eine örtliche Vorbehandlung stark verschmutzter Stellen an Textilien durchführen. Bei einer solchen örtlichen Detachur wird das Flüssig-Waschmittel auf die Zellstruktur aufgebracht und dann auf den Fleck abgedrückt. Die Übertragung erfolgt durch mechanisches Einarbeiten, wobei die Folienseite der Hand zugekehrt ist. Damit wird erreicht, daß die waschaktiven Substanzen gezielt auf die verfleckten Stellen übertragen werden. Bei dieser Manipulation erfolgt kein Produktkontakt mit der Haut. Zufolge der gezielten örtlichen Detachur unter Haushaltsbedingungen läßt sich dabei eine textilschonende Fleckvorbehandlung vornehmen. Um ein Durchdrücken des zur Detaschur benutzten Mittels zu verhindern (Benetzung von Tischplatte usw.) wird ein Teil des mit Folie versehenen Vliestuches unter die Behandlungsstelle gelegt. Anschließend kann die Vorrichtung den Textilien zum Waschvorgang beigegeben werden, so daß das Behandlungsmittel vollständig wirksam wird. Weiterhin ist die vorzugsweise in Form eines Dosiertuches gestaltete Vorrichtung für kosmetische Zwecke geeignet, um Haarfärbemittel aufzutragen, Haar- oder Gesichtspflegekuren durchzuführen, Mittel für Gesichtspackungen aufzunehmen, Produkte zur Säuberung von Gesicht von Rouge oder Eyliner aufzunehmen u.s.w.. Es ist denkbar, daß ein solches Vliestuch mit entsprechenden Aktivsubstanzen getränkt in eine Dusch- oder Badehaube eingelegt wird und dann zum Übertragen der Aktivsubstanzen auf die Haare dient. Darüber hinaus ist die Vorrichtung auch einsetzbar für medizinische Zwecke. Dort eignet sie sich zum gezielten Übertrag von Ölen oder Flüssigsubstanzen, die örtlich auf Körperteile aufgebracht werden sollen. Überdies ist die Vorrichtung auch verwendbar für Reinigungsarbeiten. Mit der Vorrichtung können Putz- und Reinigungssubstanzen, Scheuermilch, Desinfektionsmittel, Möbelpolituren, Möbelöle, Lackpflegemittel u.s.w. aufgebracht werden.

Um eine besonders hohe Saugwirkung zu erzielen, ist vorzugsweise vorgesehen, daß die Zellstruktur als Vlies ausgebildet ist. Dabei kann das Vlies aus Chemiefasern oder -fäden bestehen. Ferner ist mit Vorzug auch die Verwendung von offenporigem Schaumstoff denkbar. Sodann können sehr saugfähige Gewebe und Gewirke, insbesondere Schlingenwaren, wie Frottier-oder Frotteestoffe, eingesetzt werden.

Alternativ ist nach einer Weiterbildung der Erfindung vorgesehen, daß das Vlies aus nativen oder regenerierten Zellulosefasern oder Synthesefasern besteht, die punktförmig miteinander verbunden und somit verfestigt sind.

Die Anordnung kann so getroffen sein, daß das Vlies ein verfestigtes Faservlies ist oder aus Endloskapillaren besteht.

Die Vlieshöhe liegt bei 2 bis 30mm, vorzugsweise zwischen 2 und 10mm. Möglich sind jedoch auch Vliese unter 2mm bzw. über 30mm Vlieshöhe. Es besteht die Möglichkeit, die Zellstruktur, insbesondere mit thermisch fixierten Dosierfelder begrenzenden Einprägungen zu versehen, wodurch sich genaue Dosierungen erzielen lassen.

Die flüssigkeitsundurchlässige Folie ist insbesondere als flexible Kunststoffolie ausgebildet. Es ist möglich, Zellstruktur und Folie getrennt zu fertigen und dann miteinander zu verbinden. Alternativ kann jedoch auch vorgesehen sein, daß die Folie von einer Beschichtung oder Haut der Zellstruktur gebildet ist. Der Einsatz der Folie, Beschichtung oder Haut führt jeweils zu einer einseitigen Verfestigung der Zellstruktur, so daß diese gegenüber mechanischen Beanspruchungen widerstandsfähiger wird.

Um auch nach mehrmaligem Waschvorgang ein Loslösen der Folie von der Zellstruktur zu unterbinden, ist nach einer Weiterbildung der Erfindung vorgesehen, daß Zellstruktur und Folie randseitig miteinander vernäht sind. Besonders vorteilhaft ist es dabei, wenn die Naht als Zick-Zacknaht ausgebildet ist, da sie elastische Eigenschaften besitzt.

Alternativ oder zusätzlich ist vorgesehen, daß Folie und Zellstruktur durch eine durch Anschmelzen der Folie und/oder der Zellstruktur erzeugte Schmelzverbindung aneinander befestigt sind. Insbesondere ist es möglich, die Folie zu extrudieren und auf die noch nicht verfestigte Oberfläche der Zellstruktur aufzudrücken, so daß sich beide Teile flächig miteinander verbinden.

Nach einer weiteren Ausführungsform werden Folie und Zellstruktur flächig mit einem geeigneten Kleber miteinander verklebt. Zusätzlich kann dann noch ein Vernähen erfolgen.

Nach einer Ausgestaltung der Erfindung ist es möglich, den Träger in Beutelform zu bringen. Diese läßt sich dadurch erzielen, indem runde oder eckige Lagen mit Zugfäden ausgestattet werden. Durch Zusammenziehen solcher Zugfäden erhält man einen Beutel. Wird die Außenseite des Beutels von der Folie gebildet, so ist es neben der Dosierung von flüssigem Behandlungsmittel möglich, auch Pulver zu dosieren. Soll ein derart gefüllter Beutel dem Waschprozeß zugegeben werden, so ist es naheliegend, daß der Beutel nicht vollständig geschlossen wird, so daß das Wäschebehandlungsmittel nach und nach in die Waschlauge gelangt.

Eine Variante zeichnet sich dadurch aus, daß der Beutel Fallschirmform besitzt. Aus seiner ebenen Erstreckung wird der Fallschirm durch Zugschnüre gebracht. Zur Bildung des Fallschirmbeutels laufen die Zugschnüre durch einen Verschlußring reibungsschlüssig hindurch.

Eine andere Möglichkeit wird durch eine Waschhandschuhform des Trägers geschaffen, wobei die Außenseite des Waschhandschuhs von der Zellstruktur gebildet ist. Z.B. bei einer Fleckenvorbehandlung ist ein solcher als Waschhandschuh gestalteter Träger bequemer als ein Tuch zu handhaben.

Versuche haben gezeigt, daß es neben einer großen Speicherfähigkeit und haltbaren Ausgestaltung von Vorteil ist, wenn das Vlies aus Polyesterfasern besteht. Als Folie eignet sich insbesondere Polyethylen. Hierbei bietet sich der Einsatz von Hochdruck- bzw. Niederdruck-Polyethylen an. Eine solche Kombination zeichnet sich durch eine große Haltbarkeit aus. Mindestens zwölf Wäschen widersteht eine solche Vorrichtung, ohne daß Auflöseerscheinungen auftreten. Es wäre jedoch auch möglich, das Vlies aus Polyester- oder Polyamid- oder Polyacrylfasern und die Folie aus Polyester, Polypropylen, Polyamid, Polyurethan oder Polytetrafluorethylen herzustellen. Für verschiedene Einsatzbereiche ist eine solche Kombination günstig.

Eine optimale Verbindung von Vlies und Folie erreicht man durch ein Verschmelzen und/oder Verschweißen der beiden miteinander. Insbesondere bietet sich dabei eine Randverschweißung an.

Für viele Einsatzzwecke ist es von Vorteil, wenn das Gewicht des Vlieses 80-200g/qm beträgt.

Die Handhabung der beschriebenen Vorrichtung kann dadurch verbessert und im Gebrauchswert hinsichtlich längerer Haltbarkeit gesteigert werden, wenn die Zellstruktur (Vlies) durch eine Abdeckung gegenüber mechanischen Einflüssen geschützt wird. Die Abdeckung ist auf der der Folie gegenüberliegenden Seite des Trägers in einer die Zellstruktur überfangenden Lage angeordnet.

Vorzugsweise kann als Abdeckung ein Gewirke oder Gewebe eingesetzt werden. Alternativ ist es jedoch auch möglich ein gelochtes Flächenmaterial, insbesondere eine Lochfolie zu verwenden.

Als weitere Möglichkeit bietet sich gelochtes oder ungelochtes, thermoplastisches Fasermaterial als Abdeckung an.

Die spezielle Ausgestaltung kann so getroffen sein, daß die Abdeckung aus einem ungelochten oder gelochten Vlies, Gewebe oder Gewirke aus Polypropylen-, Polyethylen-, Polyamid-, Polyester-, Polyacryl-, Acetat- oder Polyvinylchloritfasern besteht.

Um eine festen Zusammenhalt zu schaffen, können Abdeckung und Folie miteinander verschweißt sein, was insbesondere am Rande erfolgt, jedoch auch z.B. in streifenförmigen, über die Oberfläche verteilten Zonen erfolgen kann.

Insbesondere ist es möglich, Abdeckung, Zellstruktur und Folie miteinander randzuverschweißen. Nach einer besonderen Ausführungsform besteht zwischen der Zellstruktur und der Folie ein ganzflächiger Verbund, während die Abdeckung nur am Rande mit Zellstruktur und Folie verbunden, insbesondere verschweißt ist. Dementsprechend besteht zwischen Zellstruktur und Abdeckung außerhalb der Randzonen keine Verbindung.

Die Verwendung der Vorrichtung zeichnet sich dadurch aus, daß mittels dieser das manuelle Aufbringen eines flüssigen oder pastösen Behandlungsmittels auf eine Behandlungsstelle möglich ist. Die Folie erfüllt dabei eine Doppelfunktion: Einerseits dient sie dazu, den Durchtritt des flüssigen oder pastösen Behandlungsmittels zu verhindern. Andererseits stellt sie die Fläche dar, an der die Betätigungshand, ohne in Kontakt mit dem Behandlungsmittel zu kommen, an der Vorrichtung angreifen kann. Der Einsatzbereich der Vorrichtung ist mannigfaltig, wie es eingangs schon erläutert wurde.

Schließlich ist durch die Erfindung noch ein Verfahren zum manuellen Aufbringen eines flüssigen oder pastösen Behandlungsmittels auf eine Behandlungsstelle angegeben, wobei das Behandlungsmittel auf einen saugfähigen, flexiblen Träger aufgebracht und durch eine Relativbewegung auf die Behandlungsstelle übertragen wird. Gegenüber einer solchen Aufbringungsart unterscheidet sich das erfindungsgemäße Verfahren dadurch, daß das Behandlungsmittel auf den rückseitig mit einer flüssigkeitsundurchlässigen Folie versehenen Träger aufgebracht wird und die Auftragungshand auf der dem Träger abgewandten Seite der Folie angreift. Trotz der Relativbewegung zwischen dem Träger und der Behandlungsstelle (vorzugsweise durch Auflegen bzw. Abdrücken des das Behandlungsmittel aufweisenden Trägers auf die Behandlungsstelle) gelangt die Auftragungshand nicht in Kontakt zum Behandlungsmittel. Die Relativbewegung beispielsweise bei einem Detachieren kann das Abdrücken und anschließende Reiben sein, wobei genügend Behandlungsmittel freigegeben wird. Stets ist jedoch weitgehend der Hautkontakt zum Behandlungsmittel verhindert.

Eine Fortbildung des Verfahrens ist dadurch gekennzeichnet, daß nach dem Auftragungsvorgang der mit Folie versehene Träger und die behandelte Textilie in eine Waschmaschine eingelegt werden und daß anschließend der Waschvorgang, gegebenenfalls mit zusätzlichen, behandelten oder unbehan delten weiteren Wäschestücken, durchgeführt wird. Auf diese Art und Weise wird das eingesetzte Behandlungsmittel, insbesondere Flüssigwaschmittel, doppelt genutzt, nämlich zum einen bei der Vorreinigung (Detachur) und zum anderen beim nachfolgenden Waschvorgang. Demgemäß erfordert der Waschvorgang also keine erneute Waschmittelzugabe, denn die sich in den Textilien befindliche Behandlungsmenge löst sich während des Waschvorganges und geht in die Lauge über. Dieses geschieht gleichfalls mit dem Anteil, der sich in dem Träger befindet.

Die Zeichnung veranschaulicht die Erfindung anhand von fünf Ausführungsbeispielen. Es zeigt:

Fig. 1 eine perspektivische Ansicht der erfindungsgemäßen Vorrichtung gemäß der ersten Ausführungsform,

Fig. 2 einen Querschnitt durch einen Teilabschnitt der Vorrichtung gemäß Fig. 1,

Fig. 3 einen Längsschnitt durch den in Beutelform gebrachten Träger gemäß der zweiten Ausführungsform,

Fig. 4 eine perspektivische Darstellung gemäß der dritten Ausführungsform,

Fig. 5 eine Folgedarstellung der Fig. 4, wobei der Träger in eine Fallschirmform gebracht worden ist,

Fig. 6 eine perspektivische Darstellung der Vorrichtung gemäß der vierten Ausführungsform, wobei der Träger die Gestalt eines Waschhandschuhs besitzt,

Fig. 7 eine perspektivische Darstellung der Vorrichtung gemäß der fünften Ausführungsform, welche eine Abdeckung der Zellstruktur aufweist und

Fig. 8 einen Querschnitt durch einen Teilabschnitt der Vorrichtung gemäß Fig. 7.

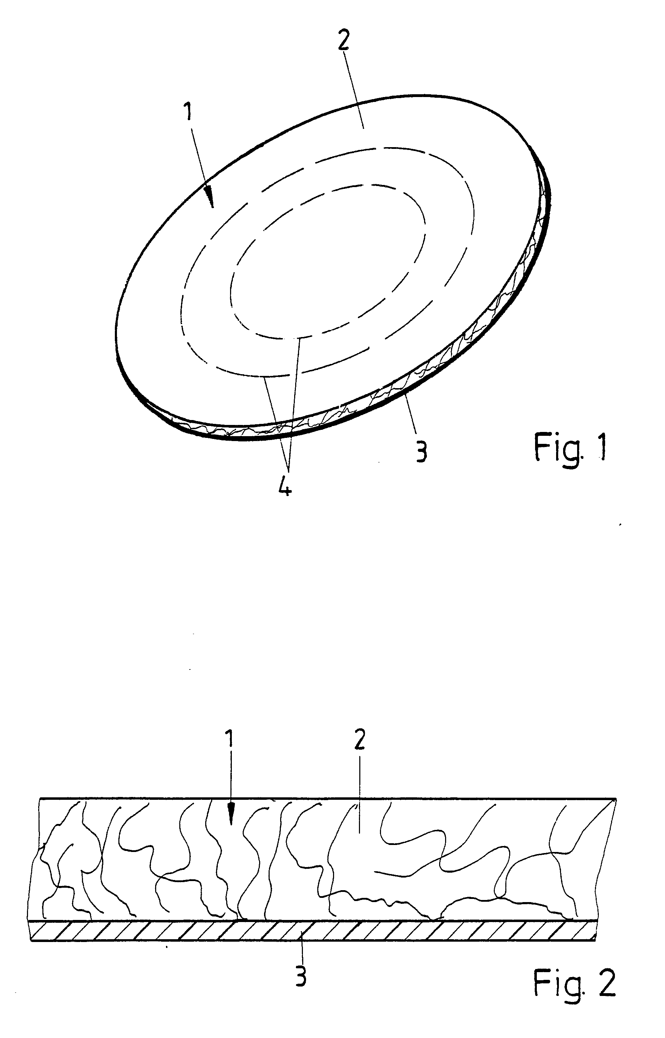

Gemäß dem ersten in den Fig. 1 und 2 aufgezeigten Ausführungsbeispiel weist die Vorrichtung eine flächige Lage einer saugfähigen, offenporigen Zellstruktur 1 auf, die als Vlies 2 ausgebildet ist und eine Kreisform besitzt. Es liegt jedoch auch im Rahmen der Erfindung, eine andere Formgestaltung zu wählen; so sind ebenfalls auch quadratische, rechteckige, vieleckige oder ovale sowie verschiedenartig figurierte Vliese 2 denkbar.

Einseitig ist das Vlies 2 mit einer flüssigkeits undurchlässigen Folie 3 versehen. Diese Undurchlässigkeit der verwendeten Folie gegenüber Flüssigkeiten ist erfindungswesentlich, denn nur so kann die Zellstruktur 1 ohne Flüssigkeitsdurchtritt bei der Aufnahme von flüssigem Behandlungsmitteln arbeiten.

Die Anwendung bei einem Waschprozeß ist folgende:

Die Vorrichtung wird mit ihrer Folie 3 nach unten auf eine vorzugsweise ebene Unterlage gelegt, und dann wird auf die Zellstruktur 1 bzw. Vlies 2 möglichst zentral flüssiges Wäschebehandlungsmittel - insbesondere Flüssig-Waschmittelgegeben, wodurch aufgrund der Saugwirkung des Vlieses 2 eine Bindungswirkung eintritt. Ein Durchtritt des flüssigen Wäschebehandlungsmittels durch das Vlies 2 wird insbesondere im Bereich anfänglich hoher Flüssigkeitskonzentrationen (z.B. im Gießkegelbereich) durch die Folie 3 verhindert. Ist der Dosiervorgang beendet, so wird sich aufgrund der Kapillarwirkung des Vlieses eine im wesentlichen gleichmäßige Flüssigkeitskonzentration über die benetzte Vliesfläche einstellen.

Das auf das Vlies 2 aufgebrachte Wäschebehandlungsmittel wird sich als kreisförmiger Fleck abzeichnen. Es ist daher möglich, die Zellstruktur 1 mit thermisch fixierten, Dosierfelder begrenzenden Einprägungen 4 zu versehen, wobei eine dieser Einprägungen entsprechende Fleckgröße des Wäschebehandlunsmittels einer bestimmten Dosiermenge entspricht.

Die Verwendung eines hochflorigen Vlieses 2 einer Größe, die kleiner als die Befüllöffnung der Waschmaschine ist, ermöglicht ohne weiteres die Bindung einer Flüssigkeitsmenge von ca. 80 - 200ml, wobei selbst nach Senkrechthalten der Vorrichtung keine Flüssigkeit austritt. Vorzugsweise liegt die Vlieshöhe zwischen 2 und 10mm.

Neben der Möglichkeit, die wasserundurchlässige Folie 3 mit Dosierangaben zu versehen, können hier auch Gebrauchshinweise oder Werbeaussagen aufgedruckt sein. Ebenfalls eignet sich das Vlies 2 für die Fixierung von Schriftzügen.

Bei Verwendung entsprechender Materialien läßt sich die Vorrichtung oftmals wiederverwenden und ist auch gegenüber Kochtemperaturen beständig. Beim Trocknen im Haushaltstrockner lassen sich die Vlieseigenschaften durch Auflockern der Fasern besonders gut erhalten.

Die Fig. 2 verdeutlicht den Aufbau des Erfindungsgegenstandes im Querschnitt. Deutlich ist erkennbar, daß die als Vlies 2 ausgebildete Zellstruktur 1 einseitig mit der Folie 3 beschichtet ist.

Nach einem nicht dargestellten Ausführungsbeispiel kann ferner vorgesehen sein, daß das Vlies 2 mit der Folie 3 randseitig vernäht ist. Die Naht ist bevorzugt als Zick-Zacknaht ausgebildet. Auch Verklebungen und Verschweißungen zur Stabilisierung des Randes sind möglich.

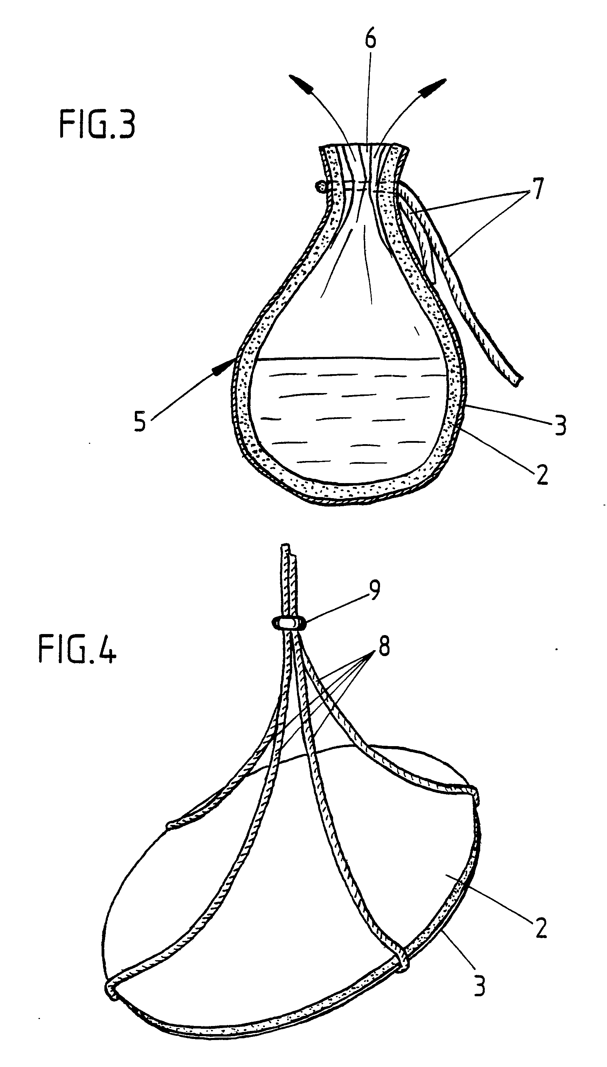

Gemäß dem zweiten, in Fig. 3 aufgezeigten Ausführungsbeispiels ist der Träger zu einem Beutel 5 geformt. Der Träger setzt sich ebenfalls aus einem Vlies 2 und einer Folie 3 zusammen. Der Beutel 5 ist so gestaltet, daß seine Außenseite von der Folie 3 gebildet ist. Der Beutelöffnung 6 ist eine Zugschnur 7 zugeordnet. Letztere läßt es zu, die Beutelöffnung 6 zum Beschicken weit zu öffnen. Gemäß dieser Ausgestaltung kann das Speichervolumen des Trägers größer sein als dasjenige der Zellstruktur. Ferner erlaubt es diese Ausgestaltung, daß sowohl flüssiges als auch pulverförmiges Behandlungsmittel aufgenommen werden kann. Nach Auffüllen des Beutels 5 mit dem Wäschebehandlungmittel wird mittels der Zugschnur 7 die Beutelöffnung 6 so weit geschlossen, daß aus ihr Behandlungsmittel austreten kann, wenn der Beutel einem Waschprozeß zugegeben wird.

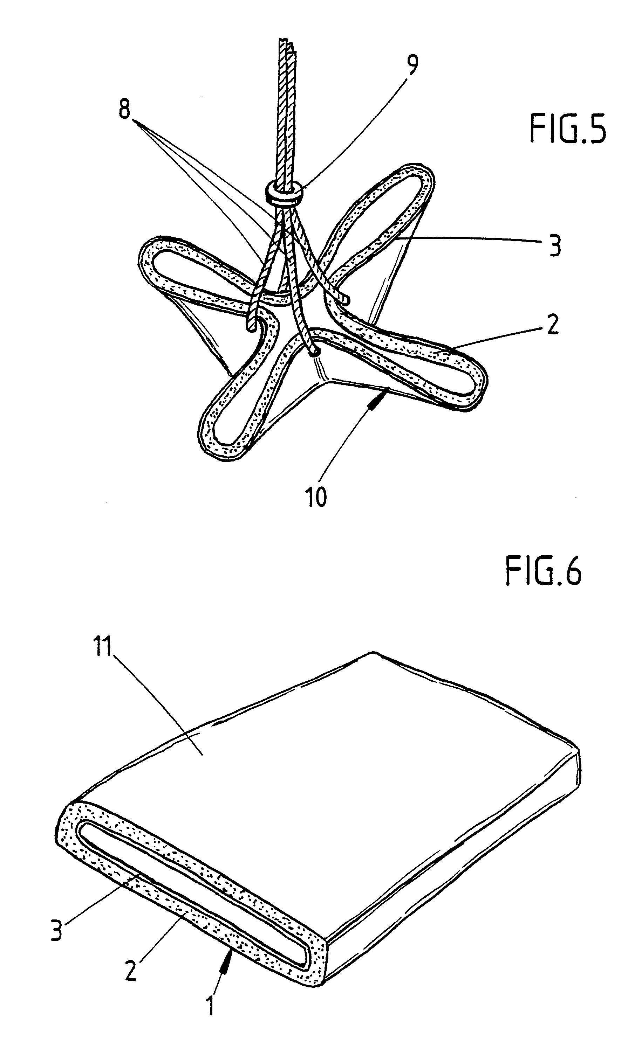

Bei der in den Fig. 4 und 5 aufgezeigten Ausgestaltung gelangt ein Vlies 2 zum Einsatz, welches dem in Fig. 1 dargestellten entspricht. Dem Vlies 2 ist ebenfalls eine flüssigkeitsundurchlässige Folie 3 zugeordnet. In gleicher Winkelverteilung gehen vom Rand des kreisförmigen Vlieses vier Zugschnüre 8 aus, die reibungsschlüssig durch einen Verschlußring 9 hindurchlaufen. Wird der Ring 9 relativ in Richtung auf das Vlies 2 verlagert, so stellt sich eine Fallschirmform gemäß Fig. 5 ein. In den Innenraum des eine Fallschirmform besitzenden Beutels 10 ist das Behandlungsmittel einzufüllen, sei es ein flüssiges oder pulverförmiges. Auch hier ist wie bei dem vorgeschilderten Ausführungsbeispiel eine solche Anordnung getroffen, daß nach Bildung der Fallschirmform des Beutels 10 die Folie 3 außenseitig liegt. Ein entsprechend gefüllter Beutel kann dann, wie vorstehend ausgeführt wurde, dem Waschvorgang zugegeben werden.

Gemäß dem in Fig. 6 veranschaulichten vierten Ausführungsbeispiel besitzt der Träger die Form eines Waschhandschuhs 11. Der Träger setzt sich ebenfalls aus einem Vlies 2 und einer Folie 3 zusammen. Im Gegensatz zur Beutelform befindet sich die Zellstruktur 1 jedoch auf der Außenseite des Waschhandschuhs 11. Ein solcher eignet sich insbesondere zur Fleckvorbehandlung. Die innenseitig des Waschhandschuhs 11 befindli che Folie 3 verhindert, daß auf die Zellstruktur 1 aufgebrachtes Behandlungsmittel in Hautkontakt mit einer in den Waschhandschuh eingeschobenen Hand gelangt. Nach einer entsprechenden Fleckvorbehandlung von Textilien kann der noch getränkte Waschhandschuh mit den Textilien dem Waschvorgang ausgesetzt werden, wobei die restliche Abgabe des Behandlungsmittels erfolgt. Auf diese Weise gelangt das Behandlungsmittel verlustfrei zur Wirkung.

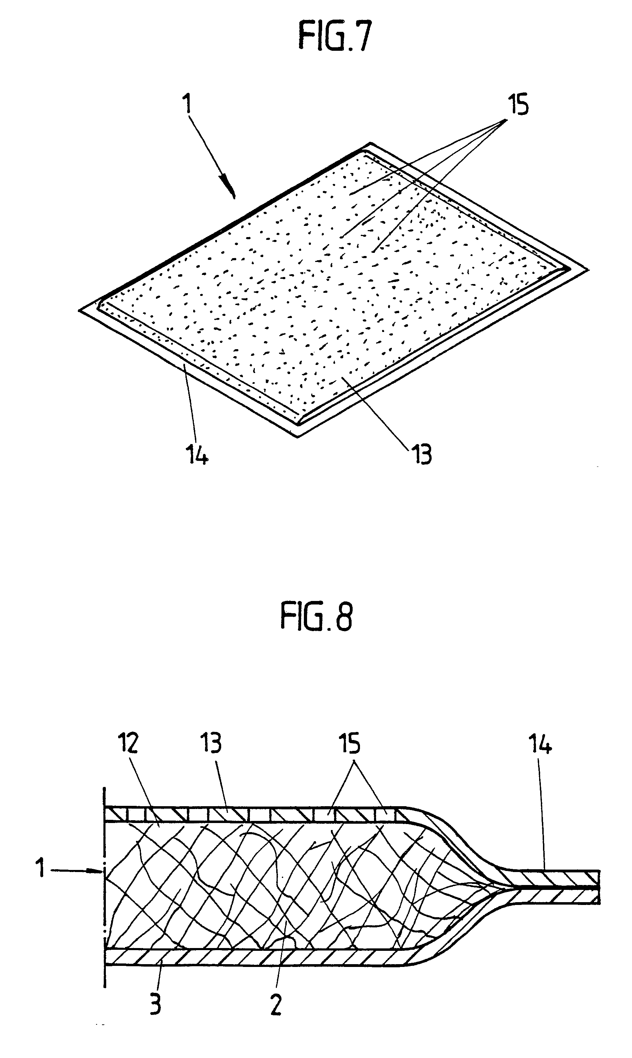

Die Fig. 7 veranschaulicht ein fünftes Ausführungsbeispiel, bei dem der Träger auf seine der Folie 3 gegenüberliegenden Seite 12 eine die Zellstruktur 1 überfangende, flüssigkeitsdurchlässige Abdeckung 13 aufweist. Demgemäß liegt gewissermaßen eine Sandwich-Bauweise vor, bestehend aus Folie 3, Zellstruktur 1 (Vlies) und Abdeckung 13. Durch die Abdeckung 13 wird die Handhabung verbessert und die Vorrichtung auch im Gebrauchswert hinsichtlich längerer Haltbarkeit gesteigert. Die Abdeckung bildet einen Schutz vor mechanischen Einflüssen.

Im Ausführungsbeispiel der Fig. 7 und 8 ist insbesondere so vorgegangen, daß die Folie 3 mit der als Vlies 2 ausgebildeten Zellstruktur 1 ganzflächig verbunden ist. Diese Verbindung kann beispielsweise durch Klebung erfolgen. Die Abdeckung 13 hingegen ist lediglich im Randbereich 14 festgelegt. Insbesondere ist hier so vorgegangen, daß Abdeckung 13, Vlies 2 und Folie 3 im Randbereich 14 miteinander verschweißt sind.

Die Abdeckung 13 ist mit einer Vielzahl von Löchern 15 versehen. Hierdurch wird diese flüssigkeitsdurchlässig. In dem dargestellten Ausführungsbeispiel besteht die Abdeckung aus gelochtem, thermoplastischem Fasermaterial.

Nach anderen, nicht dargestellten Ausführungsbeispielen ist es jedoch aus möglich die flüssigkeitsdurchlässige Abdeckung aus einem ungelochten oder gelochten Vlies, Gewebe oder Gewirke aus Polypropylen-, Polyethylen-, Polyamid-, Polyester-, Polyacryl-, Acetat- oder Polyvinylchloritfasern zu fertigen.

Die vorbeschriebenen Vorrichtungen sind nicht nur einsetzbar bei einem Waschprozeß. Vielmehr ist es auch mit ihnen möglich, ein manuelles Aufbringen eines flüssigen oder pastösen Behandlungsmittels auf eine Behandlungsstelle vorzunehmen. Die Vorrichtung ist dann verwendbar bei einem Detachieren. Ferner können kosmetische und medizinische Behandlungen sowie Reinigungsarbeiten mit Erfolg durchgeführt werden.

Durch die Erfindung ist sodann ein Verfahren angegeben, das sich zum manuellen Aufbringen eines flüssigen oder pastösen Behandlungsmittels auf eine Behandlungsstelle eignet. Das Behandlungsmittel wird auf den saugfähigen, flexiblen Träger aufgebracht und dann durch eine Relativbewegung auf die Behandlungsstelle übertragen. Da der Träger rückseitig mit der flüssigkeitsundurchlässigen Folie 3 versehen ist, scheidet weitgehend ein Hautkontakt der Auftragungshand und dem Behandlungsmittel aus, wie es vorstehend schon ausgeführt wurde. Bei einem üblichen Auftragen von Behandlungsmittel mittels eines Tuches z.B. ist dieses nicht der Fall. Sobald das Behandlungsmittel das Tuch durchdringt, gelangt es zur Betätigungshand.

Alle in der Beschreibung erwähnten und in der Zeichnung dargestellten neuen Merkmale sind erfindungswesentlich, auch soweit sie in den Ansprüchen nicht ausdrücklich beansprucht sind.

## Ansprüche

1. Vorrichtung zur Aufnahme und Abgabe eines Behandlungsmittels, bestehend aus einem das Behandlungsmittel aufnehmenden Träger, dadurch gekennzeichnet, daß der als Speicher ausgebildete Träger mindestens eine Lage einer einseitig mit einer flüssigkeitsundurchlässigen Folie (3) versehenen offenporigen, flexiblen Zellstruktur (1) aufweist.

2. Vorrichtung, insbesondere nach Anspruch 1, dadurch gekennzeichnet, daß die Zellstruktur (1) als Vlies (2) ausgebildet ist.

3. Vorrichtung, insbesondere nach Anspruch 1 und/oder 2, dadurch gekennzeichnet, daß das Vlies (2) aus Natur und/oder Chemiefasern besteht.

4. Vorrichtung, insbesondere nach Anspruch 1 und/oder 2, dadurch gekennzeichnet, daß das Vlies (2) aus nativen oder regenerierten Zellulosefasern oder Synthesefasern besteht.

5. Vorrichtung, insbesondere nach einem oder mehreren der vorhergehenden Ansprüche, dadurch gekennzeichnet, daß das Vlies (2) ein verfestigtes Faservlies ist.

6. Vorrichtung, insbesondere nach einem oder mehreren der vorhergehenden Ansprüche, dadurch gekennzeichnet, daß das Vlies Endloskapillaren aufweist.

7. Vorrichtung, insbesondere nach einem oder mehreren der vorhergehenden Ansprüche, dadurch gekennzeichnet, daß das Vlies (2) eine Höhe zwischen 2 bis 30mm, bevorzugt 2 bis 10mm aufweist.

8. Vorrichtung, insbesondere nach einem oder mehreren der vorhergehenden Ansprüche, dadurch gekennzeichnet, daß die Zellstruktur (1) insbesondere mit thermisch fixierten, Dosierfelder begrenzenden Einprägungen versehen ist.

9. Vorrichtung, insbesondere nach einem oder mehreren der vorhergehenden Ansprüche, dadurch gekennzeichnet, daß die Folie (3) eine flexible Kunststoffolie ist.

10. Vorrichtung, insbesondere nach einem oder mehreren der vorhergehenden Ansprüche, dadurch gekennzeichnet, daß die Folie (3) als eine Beschichtung oder Haut der Zellstruktur (1) ausgebildet ist.

11. Vorrichtung, insbesondere nach einem oder mehreren der vorhergehenden Ansprüche, dadurch gekennzeichnet, daß Zellstruktur (1) und Folie (3) randseitig miteinander vernäht sind.

12. Vorrichtung, insbesondere nach Anspruch 11, dadurch gekennzeichnet, daß die Naht als Zick-Zacknaht ausgebildet ist.

13. Vorrichtung, insbesondere nach einem oder mehreren der vorhergehenden Ansprüche, dadurch gekennzeichnet, daß Folie (3) und Zellstruktur (1) durch eine durch Anschmelzen der Folie (3) und/oder der Zellstruktur (1) erzeugte Schmelzverbindung aneinander befestigt sind.

14. Vorrichtung, insbesondere nach einem oder mehreren der vorhergehenden Ansprüche, dadurch gekennzeichnet, daß Folie (3) und Zellstruktur (1) miteinander verklebt sind.

15. Vorrichtung, insbesondere nach einem oder mehreren der vorhergehenden Ansprüche, gekennzeichnet durch eine Beutelform des Trägers.

16. Vorrichtung, insbesondere nach Anspruch 15, dadurch gekennzeichnet, daß die Außenseite des Beutels (5, 10) von der Folie (3) gebildet ist.

17. Vorrichtung, insbesondere nach einem oder mehreren der vorhergehenden Ansprüche, dadurch gekennzeichnet, daß der Beutel (10) Fallschirmform besitzt.

18. Vorrichtung, insbesondere nach Anspruch 17, dadurch gekennzeichnet, daß die Zugschnüre (8) des Fallschirmbeutels (10) durch einen Verschlußring (9) reibungsschlüßig hindurchlaufen.

19. Vorrichtung, insbesondere nach einem oder mehreren der vorhergehenden Ansprüche, gekennzeichnet durch eine Waschhandschuhform des Trägers, wobei die Außenseite des Waschhandschuhs (11) von der Zellstruktur (1) gebildet ist.

20. Vorrichtung, insbesondere nach einem oder mehreren der vorhergehenden Ansprüche, dadurch gekennzeichnet, daß das Vlies (2) aus Synthesefasern, bevorzugt Polyesterfasern besteht.

21. Vorrichtung, insbesondere nach einem oder mehreren der vorhergehenden Ansprüche, dadurch gekennzeichnet, daß die Folie (3) aus einem flexiblen thermoplastischen Kunststoff, bevorzugt aus Polyester oder Polyethylen oder Polyurethan besteht.

22. Vorrichtung, insbesondere nach einem oder mehreren der vorhergehenden Ansprüche, dadurch gekennzeichnet, daß Vlies (2) und Folie (3) miteinander verschmolzen und/oder verschweißt und/oder verklebt sind.

23. Vorrichtung, insbesondere nach einem oder mehreren der vorhergehenden Ansprüche, dadurch gekennzeichnet, daß Vlies (2) und Folie (3) miteinander randverschweißt sind.

24. Vorrichtung, insbesondere nach einem oder mehreren der vorhergehenden Ansprüche, dadurch gekennzeichnet, daß das Gewicht des Vlieses 80-200g/qm beträgt.

25. Vorrichtung, insbesondere nach einem oder mehreren der vorhergehenden Ansprüche, dadurch gekennzeichnet, daß der Träger auf seiner der Folie (3) gegenüberliegenden Seite (12) eine die Zellstruktur (1) überfangende, flüssigkeitsdurchlässige Abdeckung (13) aufweist.

26. Vorrichtung, insbesondere nach Anspruch 25, dadurch gekennzeichnet, daß die Abdeckung (13) aus Gewirke oder Gewebe besteht.

27. Vorrichtung, insbesondere nach Anspruch 25, dadurch gekennzeichnet, daß die Abdeckung (13) aus gelochtem Flächenmaterial, insbesondere Folie besteht.

28. Vorrichtung, insbesondere nach einem oder mehreren der vorhergehenden Ansprüche 25 - 27, dadurch gekennzeichnet, daß die Abdeckung (13) aus thermoplastischem Fasermaterial besteht.

29. Vorrichtung, insbesondere nach einem oder mehreren der vorhergehenden Ansprüche 25 - 28, dadurch gekennzeichnet, daß die Abdeckung (13) aus einem ungelochten oder gelochten Vlies, Gewebe oder Gewirke aus Polypropylen-, Polyethylen-, Polyamid-, Polyester-, Polyacryl-, Acetat- oder Polyvinylchloridfasern besteht.

30. Vorrichtung, insbesondere nach einem oder mehreren der vorhergehenden Ansprüche 25 - 29, dadurch gekennzeichnet, daß Abdeckung (13) und Folie (3) miteinander verschweißt, insbesondere randverschweißt sind.

31. Vorrichtung, insbesondere nach einem oder mehreren der vorhergehenden Ansprüche 25 - 30, dadurch gekennzeichnet, daß Abdeckung (13), Zellstruktur (1) und Folie (3) miteinander verschweißt, insbesondere randverschweißt sind.

32. Verwendung der Vorrichtung nach einem oder mehreren der vorhergehenden Ansprüche 1 - 31 zum manuellen Aufbringen eines flüssigen oder pastösen Behandlungsmittels auf eine Behandlungsstelle durch Aufdrücken und /oder Einreiben.

33. Verwendung der Vorrichtung nach einem oder mehreren der vorhergehenden Ansprüche 1 - 32 zum manuellen Aufbringen eines Wasch- oder Reinigungsmittels auf eine Textilie zur örtlichen Detachur.

34. Verwendung der Vorrichtung nach einem oder mehreren der vorhergehenden Ansprüche 1 - 32 zum manuellen Aufbringen eines kosmetischen Mittels auf Haut oder Haare.

35. Verfahren zum manuellen Aufbringen eines flüssigen oder pastösen Behandlungsmittels auf eine Behandlungsstelle, wobei das Behandlungsmittel auf einen saugfähigen, flexiblen Träger aufgebracht und durch eine Relativbewegung auf die Behandlungsstelle übertragen wird, dadurch gekennzeichnet, daß das Behandlungsmittel auf den rückseitig mit einer flüssigkeitsundurchlässigen Folie (3) versehenen Träger (Vlies 2) aufgebracht wird und die Auftragungshand auf der dem Träger abgewandten Seite der Folie (3) angreift.

36. Verfahren nach Anspruch 35, dadurch gekennzeichnet, daß als Behandlungsmittel ein auf eine Textilie aufzubringenden Flüssigwaschmittel verwendet wird.

37. Verfahren nach Anspruch 35 und/oder 36, dadurch gekennzeichnet, daß nach dem Auftragungsvorgang der mit Folie versehene Träger und die behandelte Textilie in eine Waschmaschine eingelegt werden und daß anschließend der Waschvorgang, gegebenenfalls mit zusätzlichen, behandelten oder auch unbehandelten weiteren Wäschestücken, durchgeführt wird.

Fig. 1

Fig. 2

FIG.3

FIG.4

FIG.5

9

8

3

2

10

FIG.6

11

3

2

1

# FIG.7

# FIG.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4254139 (COLGATE-PALMOLIVE COMPANY)<br>* das ganze Dokument * | 1-7, 9, 10, 20-24, 35-37 | D06F39/02<br>D06M21/02 |
| A | | 8, 11, 13, 14, 32-34 | |
| X | US-A-4105813 (ECONOMICS LABORATORY INC.)<br>* Spalte 4, Zeile 25 - Spalte 5, Zeile 38; Figuren 2, 3 * | 1-3, 25-29 | |
| A | | 4-7, 10-14, 20, 22-24, 30, 31 36, 37 | |

| RECHERCHIERTE SACHGEBIETE (Int. Cl.4) |
|---|
| D06F<br>D06M |

Der vorliegende Recherchenbericht wurde für alle Patentansprüche erstellt

| Recherchenort | Abschlußdatum der Recherche | Prüfer |
|---|---|---|
| DEN HAAG | 19 APRIL 1989 | COURRIER G.L.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................
& : Mitglied der gleichen Patentfamilie, übereinstimmendes Dokument

EPO FORM 1503 03.82 (P0403)